(19) Europäisches Patentamt
European Patent Office
Office européen des brevets

(11) **EP 4 495 564 A1**

(12) **EUROPEAN PATENT APPLICATION**
published in accordance with Art. 153(4) EPC

(43) Date of publication:
**22.01.2025 Bulletin 2025/04**

(21) Application number: **23770801.1**

(22) Date of filing: **14.03.2023**

(51) International Patent Classification (IPC):
*G01L 1/16* [(2006.01)]       *A61B 5/11* [(2006.01)]
*A61B 5/113* [(2006.01)]

(52) Cooperative Patent Classification (CPC):
**A61B 5/11; A61B 5/113; G01L 1/16**

(86) International application number:
**PCT/JP2023/009915**

(87) International publication number:
**WO 2023/176847 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.03.2022   JP 2022044701**

(71) Applicant: **Mitsubishi Chemical Corporation
Tokyo 100-8251 (JP)**

(72) Inventor: **WATANABE Itaru
Tokyo 100-8251 (JP)**

(74) Representative: **Hoffmann Eitle
Patent- und Rechtsanwälte PartmbB
Arabellastraße 30
81925 München (DE)**

(54) **ELECTRONIC APPARATUS, BED SENSOR, AND SENSOR FOR SHELF**

(57)     An electronic apparatus (1) includes a sensor (10) that is a piezoelectric element-type sensor which outputs a voltage corresponding to an applied pressure, and a first detection circuit (20) that detects a continuous pressure applied to the sensor (10) from the voltage output from the sensor (10). The first detection circuit (20) has a capacitor (21) that is connected in parallel to the sensor (10) and a high-input impedance circuit (22) that is connected in series to the sensor (10).

FIG. 1

**Description**

[Technical Field]

The present invention relates to an electronic apparatus, a bed sensor, and a sensor for a shelf.

Priority is claimed on Japanese Patent Application No. 2022-044701, filed March 18, 2022, the content of which is incorporated herein by reference.

[Background Art]

There are various types of so-called pressure sensors that convert information relating to a pressure into an electric signal, and various types of sensing using physical quantities such as light, magnetostriction, string vibration, electrostatic capacitance, inductance, and the like have been studied.

For example, Patent Documents 1 to 3 propose techniques for detecting respiration, pulse rates, body movement, and the like using piezoelectric films such as PVDF films , porous polypropylene electret film, and the like.

A sensor using a piezoelectric film such as a PVDF film, a porous polypropylene electret film, or the like is also referred to as a piezoelectric element-type sensor, and detects a voltage generated by a pressure applied to the sensor. Since the piezoelectric element-type sensor generally outputs a pulsed signal at the timing at which a pressure change occurs, it is possible to obtain information (hereinafter, also referred to as "dynamic information") about such a stimulus by which the pressure or the load changes. However, it has been considered difficult to obtain information (hereinafter, also referred to as "static information") about such a stimulus by which a certain pressure or a certain load continues to be applied.

On the other hand, Patent Document 4 proposes a technique of measuring a posture using a static acceleration sensor, and a piezo-resistive sensor is exemplified as the static acceleration sensor.

The piezo-resistive sensor detects a resistance change inside the sensor due to a pressure, and is able to acquire static information to quantify the pressure or the load. However, it is difficult to acquire dynamic information or to accurately deal with an instantaneous pressure change or load change.

Further, as a technique for quantifying the pressure or the load, Patent Document 5 proposes a technique of detecting a change in load applied to the bed by a load cell.

[Citation List]

[Patent Documents]

[Patent Document 1]
Japanese Unexamined Patent Application, First Publication No. 2001-187030
[Patent Document 2]
Japanese Unexamined Patent Application, First Publication No. 2008-93395
[Patent Document 3]
Japanese Unexamined Patent Application, First Publication No. 2010-51588
[Patent Document 4]
Japanese Unexamined Patent Application, First Publication No. 2006-271501
[Patent Document 5]
PCT International Publication No. WO2015/008677

[Summary of Invention]

[Technical Problem]

Meanwhile, in sensors for the health care field, the robotics field, and the haptics field, both dynamic information and static information may be required. For example, in the sensor for the health care field, it is required to acquire both dynamic information such as respiration, pulse rate, body movement, and the like, and static information such as a posture during sleep, a sleeping position, and the like.

In the related art, in order to acquire both the dynamic information and the static information, it is necessary to dispose a sensor in accordance with a signal to be acquired. For example, a piezoelectric element-type sensor from which the dynamic information is obtained and a piezo-resistive sensor from which the static information is obtained may be combined, and a load cell may be used.

However, in each field, there is a demand for cost reduction and space saving of the sensor, and the hybridization of a plurality of sensors is avoided. Therefore, there is a tendency that a technique is necessary which is capable of acquiring both the dynamic information and the static information with a lower cost than the load cell and without using a plurality of types of sensors.

An object of the present invention is to provide an electronic apparatus which is capable of acquiring the static information with one piezoelectric element-type sensor which is less expensive than the load cell, and a bed sensor and a sensor for a shelf that include the electronic apparatus.

[Solution to Problem]

As a result of intensive studies, the present inventors have found that static information can be acquired by using a piezoelectric element-type sensor by devising a detection circuit that detects an output voltage of the piezoelectric element-type sensor. That is, the gist of the present invention is as follows.

A first aspect of the present invention relates to an electronic apparatus comprising: a piezoelectric element-type sensor configured to output a voltage corresponding to an applied pressure; and a first detection circuit configured to detect a continuous pressure applied to the piezoelectric element-type sensor from the output voltage of the piezoelectric element-type sensor, wherein the first detection circuit has a capacitor that is connected in parallel to the piezoelectric element-type sensor, and a high-input impedance circuit that is connected in series to the piezoelectric element-type sensor.

A second aspect of the present invention relates to the electronic apparatus according to the first aspect, wherein an input impedance of the high-input impedance circuit is 0.1 T$\Omega$ or more and 10 T$\Omega$ or less.

A third aspect of the present invention relates to the electronic apparatus according to the first or second aspect, further comprising: a differential circuit configured to be connected in series to the first detection circuit and to detect a change in pressure applied to the piezoelectric element-type sensor by differentiating a detection result of the first detection circuit.

A fourth aspect of the present invention relates to the electronic apparatus according to the first or second aspect, further comprising: a second detection circuit configured to detect a change in pressure applied to the piezoelectric element-type sensor from the output voltage of the piezoelectric element-type sensor.

A fifth aspect of the present invention relates to the electronic apparatus according to the fourth aspect, wherein an input impedance of the high-input impedance circuit included in the first detection circuit is higher than an input impedance of the second detection circuit.

A sixth aspect of the present invention relates to the electronic apparatus according to the fifth aspect, wherein the input impedance of the high-input impedance circuit included in the first detection circuit is 104 times or more and 108 times or less than the input impedance of the second detection circuit.

A seventh aspect of the present invention relates to the electronic apparatus according to the fourth to sixth aspects, wherein an input impedance of the second detection circuit is 0.1 T$\Omega$ or more and 10 T$\Omega$ or less.

An eighth aspect of the present invention relates to the electronic apparatus according to any one of the fourth to seventh aspects, further comprising: a switching circuit configured to perform switching as to whether to connect the first detection circuit or to connect the second detection circuit to the piezoelectric element-type sensor.

A ninth aspect of the present invention relates to the electronic apparatus according to any one of the first to eighth aspects, wherein an electrostatic capacitance of the capacitor is 100 pF or more and 5000 pF or less.

A tenth aspect of the present invention relates to the electronic apparatus according to any one of the first to ninth aspects, wherein the piezoelectric element-type sensor includes an electret film.

An eleventh aspect of the present invention relates to the electronic apparatus according to the tenth aspect, wherein the electret film contains a polyolefin-based resin.

A twelfth aspect of the present invention relates to a bed sensor comprising: the electronic apparatus according to any one of the first to eleventh aspects.

A thirteenth aspect of the present invention relates to a sensor for a shelf comprising: the electronic apparatus according to any one of the first to eleventh aspects.

[Advantageous Effects of Invention]

According to the present invention, since the static information can be acquired with one piezoelectric element-type sensor which is less expensive than a load cell, it is possible to reduce the cost of the sensor and to save space.

[Brief Description of Drawings]

FIG. 1 is a block diagram showing a configuration of a main part of an electronic apparatus according to a first embodiment of the present invention.

FIG. 2 is an exploded perspective view showing a basic configuration of a sensor used in the first embodiment of the present invention.

FIG. 3 is a block diagram showing a configuration of a main part of an electronic apparatus according to a second embodiment of the present invention.

FIG. 4 is a block diagram showing a configuration of a main part of an electronic apparatus according to a third embodiment of the present invention.

FIG. 5 is a block diagram showing a configuration of a main part of an electronic apparatus according to a fourth embodiment of the present invention.

FIG. 6 is a diagram showing a waveform of an output voltage acquired by the second detection circuit in a case where the sensor of Example 1 was used.

FIG. 7 is a diagram showing a waveform of an output voltage acquired by the first detection circuit in a case where the sensor of Example 1 was used.

FIG. 8 is a diagram showing the relationship between the magnitude of a load applied to the sensor of Example 1 and the magnitude of the output voltage of the first detection circuit.

FIG. 9 is a diagram showing a waveform of an output voltage acquired by the first detection circuit in a case where the sensor of Example 2 was used.

[Description of Embodiments]

Hereinafter, embodiments of the present invention will be described in detail. The configurations of the following embodiments are merely examples, and the present invention is not limited to the configurations of the embodiments.

[First Embodiment]

< Electronic Apparatus >

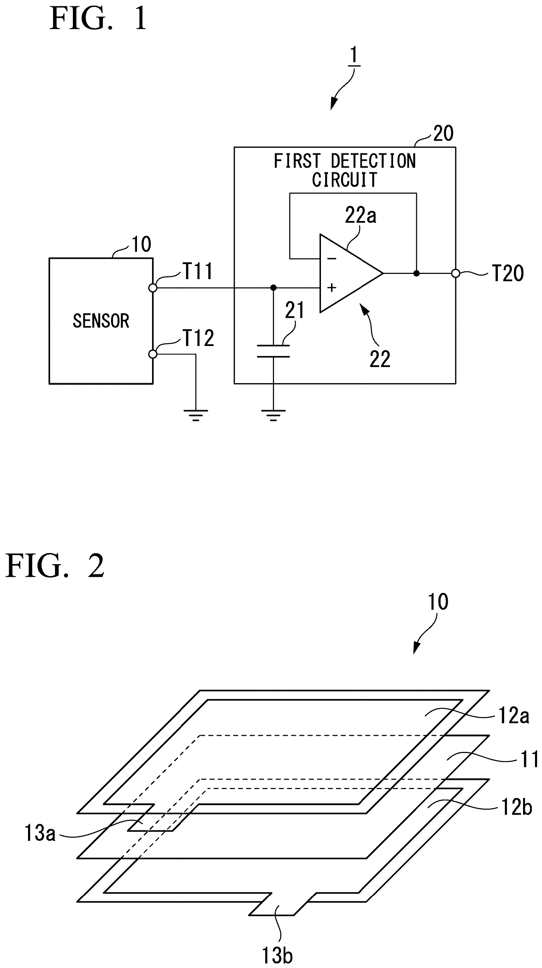

FIG. 1 is a block diagram showing a configuration of a main part of an electronic apparatus according to a first embodiment of the present invention. As shown in FIG. 1, an electronic apparatus 1 according to the present embodiment includes a sensor 10 and a first detection circuit 20, and detects static information applied to the sensor 10. Here, the static information is information relating to a stimulus by which a certain pressure or load is continuously applied. The static information can also be referred to as a continuous pressure applied to the sensor 10. Here, the continuous pressure can be referred to as a pressure or a load that does not change with time, or a pressure or a load with a small change with time. That is, it can be referred to as a pressure or a load with a change rate with respect to time is zero or can be regarded as zero.

The sensor 10 is a sensor that outputs a voltage corresponding to an applied pressure. The sensor 10 is a piezoelectric element-type sensor and outputs a pulsed signal at a timing at which a pressure change occurs. The sensor 10 includes two output terminals T11 and T12. The output terminal T11 is connected to the first detection circuit 20, and the output terminal T12 is grounded. The sensor 10 outputs the above-mentioned pulsed signal from the output terminal T11 in a case where the pressure is applied. Details of the sensor 10 will be described later.

The first detection circuit 20 includes a capacitor 21 and a high-input impedance circuit 22, and detects the static information applied to the sensor 10 from the output voltage of the sensor 10. The first detection circuit 20 may be an analog circuit or may be a digital circuit. The capacitor 21 is connected in parallel to the sensor 10. Specifically, one electrode of the capacitor 21 is connected to the output terminal T11 of the sensor 10, and the other electrode is grounded. The capacitor 21 is provided to perform charging and discharging of the charge generated in the sensor 10, thereby performing waveform control of the pulsed signal which is output from the output terminal T11 of the sensor 10. The electrostatic capacitance of the capacitor 21 is, for example, 100 pF or more and 5000 pF or less, preferably 200 pF or more and 3000 pF or less, and more preferably 500 pF or more and 2000 pF or less.

The high-input impedance circuit 22 includes an operational amplifier 22a, and is a circuit that is connected in series to the sensor 10. The high-input impedance circuit 22 is a circuit in which an inverting input terminal and an output terminal of the operational amplifier 22a are connected (so-called voltage follower circuit (buffer circuit)), and a noninverting input terminal of the operational amplifier 22a is connected to the output terminal T11 of the sensor 10. The high-input impedance circuit 22 is provided to prevent the outflow of the charge generated in the sensor 10. The input impedance of the high-input impedance circuit 22 is, for example, 0.1 TΩ or more and 10 TΩ or less, preferably 0.2 TΩ or more and 8 TΩ or less, and more preferably 0.5 TΩ or more and 5 TΩ or less.

An output terminal of the high-input impedance circuit 22 (an output terminal of the operational amplifier 22a) is connected to an output terminal T20 of the first detection circuit 20. A signal indicating the static information applied to the sensor 10 is output from the output terminal T20 of the first detection circuit 20. A circuit, such as an amplification circuit or the like that amplifies the signal which is output from the high-input impedance circuit 22, may be provided between the high-input impedance circuit 22 and the output terminal T20 of the first detection circuit 20.

< Sensor >

As described above, the sensor 10 is a piezoelectric element-type sensor. The piezoelectric element-type sensor is not particularly limited as long as the sensor is a sensor that is capable of detecting a voltage generated by a pressure applied to the sensor, and examples thereof include a sensor including a ceramics-based or organic polymer-based piezoelectric material.

Examples of the ceramics-based piezoelectric material include lead zirconate titanate (PZT), barium strontium titanate (BST), and the like.

Examples of the organic polymer-based piezoelectric material include an electret film or the like which is obtained by charging a film consisting of a permanent dipole material such as polyvinylidene fluoride (PVDF), a vinylidene fluoride and ethylene trifluoride copolymer (P(VDF-TrFE)), a vinylidene fluoride and ethylene tetrafluoride copolymer (P(VDF-TFE)), polylactic acid (PLA), or the like, and a polyolefin-based resin, and the like.

Among these materials, from the viewpoint of piezoelectric characteristics and flexibility of the sensor, an organic polymer-based piezoelectric material is preferable, and an electret film is more preferable.

(1-1) Electret Film

The type of the electret film is not particularly limited as long as the electret film has piezoelectric characteristics, but a porous electret film is preferable from the viewpoint of further enhancing the piezoelectric characteristics. Further, it is more preferable that the electret film be formed of a porous film that is used by being charged.

In a case where the porous electret film is used, a method of making the film porous is not particularly limited, and examples thereof include a method of making the film porous through chemical or physical foaming and stretching. Among the methods, from the viewpoint that a dense porous structure is obtained and the shapes of the pores are easily controlled, it is preferable that the porous structure be formed by stretching.

Examples of the material of the electret film include a polyolefin-based resin, a fluorine resin, a vinyl chloride resin, a polystyrene-based resin, a butadiene-based resin, a polyester-based resin, an acrylic-based resin, and the like. Among these resins, from the viewpoint of low environmental load and ease of performing the charging treatment, a polyolefin-based resin is suitably used.

(1-1-1) Polyolefin-Based Resin

The electret film according to the present embodiment is preferably made of a polyolefin-based resin as a main component, and more preferably a polypropylene-based resin as a main component.

In the present invention, "main component" means that the content in the electret film is 50% by mass or more, preferably 70% by mass or more, more preferably 80% by mass or more, and yet more preferably 90% by mass or more. An upper limit of the content thereof is not particularly limited, and may be 100% by mass or less.

Examples of the polypropylene-based resin include homopolypropylene (propylene homopolymer), or a random copolymer or a block copolymer of propylene and $\alpha$-olefins such as ethylene, 1-butene, 1-pentene, 1-hexene, 1-heptene, 1-octene, 1-nonene, 1-decene, or the like. Among these components, from the viewpoint of mechanical strength, homopolypropylene is more suitably used.

Further, an isotactic pentad fraction exhibiting stereoregularity of the polypropylene-based resin is preferably 80% or more, more preferably 83% or more, and yet more preferably 85% or more. In addition, the isotactic pentad fraction is preferably 99% or less, more preferably 98% or less, and yet more preferably 97% or less.

In a case where the isotactic pentad fraction is 80% or more, the mechanical strength is favorable. On the other hand, the upper limit of the isotactic pentad fraction is defined by the upper limit value currently obtained industrially, but the upper limit is not applied in a case where a resin having further higher regularity is developed at an industrial level in the future. The isotactic pentad fraction means a stereoscopic structure or a proportion of the stereoscopic structure in which five methyl groups, which are side chains with respect to a main chain composed of carbon-carbon cements in any five consecutive propylene units, are all located in the same direction. Attribution of the signals in the methyl group region is based on A. Zambelli et al. (Macromol. 8, 687 (1975)).

Further, Mw/Mn of the polypropylene-based resin, which is a parameter indicating a molecular weight distribution, is preferably 1.5 or more, more preferably 2.0 or more. In addition, Mw/Mn is preferably 10.0 or less and more preferably 8.0 or less, and yet more preferably 6.0 or less.

A smaller Mw/Mn means a narrower molecular weight distribution. However, by setting Mw/Mn to 1.5 or more, sufficient extrusion moldability is obtained. Thus, it is possible to achieve mass production on an industrial scale. On the other hand, in a case where Mw/Mn is 10.0 or less, sufficient mechanical strength can be ensured.

Mw/Mn is measured by a gel permeation chromatography (GPC) method as a polystyrene conversion value.

Further, a melt flow rate (MFR) of the polypropylene-based resin is not particularly limited. However, the melt flow rate is preferably 0.5 g/10 min or more, more preferably 1.0 g/10 min or more. In addition, the melt flow rate is preferably 15 g/10 min or less, and more preferably 10 g/10 min or less.

In a case where the MFR is 0.5 g/10 min or more, the polypropylene-based resin has a sufficient melt viscosity during a molding process, and high productivity can be ensured. In contrast, in a case where the MFR is 15 g/10 min or less, sufficient strength can be ensured.

The MFR is measured under conditions of a temperature of 230°C and a load of 2.16 kg based on JIS K7210-1 (2014).

A method for producing the polypropylene-based resin is not particularly limited, and examples thereof include a known polymerization method using a known polymerization catalyst, such as a polymerization method using a multisite catalyst typified by a Chugler-Natta type catalyst or a single site catalyst typified by a metallocene-based catalyst.

Examples of a polypropylene-based resin which can be suitably used in the present embodiment include commercially available products such as trade names "NOVATEC PP", "WINTEC", and "WAYMAX" (manufactured by Japan Polypropylene Corporation), "VERSIFY", "NOTIO", and "TAFMER XR" (manufactured by Mitsui Chemicals, Inc.), "ZERAS" and "THERMOLAN" (manufactured by Mitsubishi Chemical Corp.), "SUMITOMO NOBLEN" and "TAFTHREN" (manufactured by Sumitomo Chemical Company, Limited), "PRIME POLYPRO" and "PRIME TPO" (manufactured by Prime Polymer Co., Ltd.), "ADFLEX", "ADSYL", and "HMS-PP (PF814)" (manufactured by SunAllomer Ltd.), "INSPIRE" (manufactured by Dow Chemical), and the like.

[β-Crystal Activity]

In a case where the electret film according to the present embodiment contains a polypropylene-based resin as a main component, it is preferable that the electret film consist of a resin composition having a main component of a polypropylene-based resin that contains a large amount of β-crystals, which are one of crystal forms. A non-porous film material consisting of a resin composition containing a polypropylene-based resin containing a large amount of β-crystals as a main component exhibits excellent piezoelectricity even after the charging treatment. However, further excellent piezoelectricity is obtained by stretching the non-porous film material to have a porous structure. In the formation of the porous structure using the β-crystal, the porous structure is formed in a process in which the β-crystal in the polypropylene-based resin is transformed into the α-crystal in the stretching process. Therefore, the porous structure is dense and the preparation thereof is advantageous since the porous structure does not depend on the particle size or the dispersion diameter, as compared with the formation of a porous structure by adding an inorganic filler or an incompatible organic substance known in the related art.

In the film which is made porous by using the β-crystal, since the porous structure is dense and the surface area of the pores is large, more charge is likely to be trapped in the charging treatment. Since the porous electret film exhibits piezoelectricity due to charge trapped at an interface between the pores and a matrix, piezoelectric characteristics tend to be improved in a case where a porous structure of the film is dense. Further, in a case where the porous structure is dense, distances between the pores are extremely short, and the trapped charge is easily fixed by Coulomb force between the charges. Thereby, the trapped charge is less likely to be discharged, and characteristics as an electret film are less likely to deteriorate.

The β-crystal activity of the electret film in the present embodiment can be regarded as an indicator that the polypropylene-based resin has generated a β-crystal in the non-porous film material before stretching. In a case where the polypropylene-based resin in the non-porous film material before stretching has generated β-crystals, many fine and uniform pores are formed by performing the subsequent stretching. Therefore, mechanical characteristics are excellent, and excellent breakdown voltage resistance can be obtained by the formation of fine and uniform pores.

In the present embodiment, the presence or absence of the β-crystal activity of the electret film is determined by performing differential thermal analysis of the electret film using a differential scanning calorimeter (DSC) and detecting a crystal melting peak temperature derived from the β-crystal of the polypropylene-based resin.

Specifically, the laminated porous film is heated from 40°C to 200°C at a heating rate of 10°C/min by the differential scanning calorimeter, maintained at an elevated temperature for 1 minute, cooled from 200°C to 40°C at a cooling rate of 10°C/min, maintained at a cooled temperature for 1 minute, and reheated from 40°C to 200°C at a heating rate of 10°C/min. Then, the crystal melting peak temperature (Tmβ) derived from the β-crystal of the polypropylene-based resin is detected during the reheating. In such a case, it is determined that the laminated porous film has β-crystal activity.

The presence or absence of the β-crystal activity can also be determined from a diffraction profile obtained by the X-ray diffraction measurement of the electret film which has been subjected to a specific heat treatment. Specifically, the X-ray diffraction measurement is performed on the electret film which has been subjected to the heat treatment at a temperature of 170°C to 190°C, which is a temperature higher than a crystal melting peak temperature of the polypropylene-based resin, and has been slowly cooled to generate and grow β-crystals. Then, a diffraction peak derived from (300) planes of the β-crystals of the polypropylene-based resin is detected in a range of 2θ = 16.0° to 16.5°. In such a case, it is determined that the β-crystal activity is present. Regarding the β-crystal structure of the polypropylene-based resin and the X-ray diffraction measurement, details can be found in Macromol. Chem. 187, 643-652 (1986), Prog. Polym. Sci. Vol. 16, 361-404 (1991), Macromol. Symp. 89, 499-511 (1995), Macromol. Chem. 75, 134 (1964), and references cited in the documents.

Examples of a method for obtaining the β-crystal activity of the above-mentioned polypropylene-based resin include a method of not adding a substance that promotes the generation of the α-crystal of the polypropylene-based resin, a method of adding the polypropylene-based resin which has been treated to generate a peroxide radical as described in Japanese Patent (Granted) Publication No. 3739481, and a method of adding a β-crystallizing agent. However, in the present embodiment, the method of adding the β-crystallizing agent to obtain the β-crystal activity is particularly preferable. By adding the β-crystallizing agent, it is possible to more homogeneously and efficiently promote the generation of the β-crystal of the polypropylene-based resin, and it is possible to obtain the electret film having β-crystal activity.

The degree of the β-crystal activity can be quantified by measuring the β-crystal generative capacity. The β-crystal generative capacity of the polypropylene-based resin contained in the electret film is preferably 80% or more, more preferably 85% or more, and yet more preferably 90% or more.

In a case where the β-crystal generative capacity of the polypropylene-based resin is the above-mentioned lower limit or more, suitable piezoelectricity can be exhibited. The upper limit thereof is not particularly limited, but is usually 100% or less. The β-crystal generative capacity is calculated by a method to be described later.

(1-1-2) β-Crystallizing Agent

In a case where the electret film according to the present embodiment contains a polypropylene-based resin as a main component, in order to obtain excellent piezoelectricity, it is preferable that the electret film contain the β-crystallizing agent. By containing the β-crystallizing agent in the electret film, it is possible to obtain the β-crystal activity. Examples of the β-crystallizing agent used in the present embodiment include β-crystallizing agents shown below. The β-crystallizing agent may be used alone or in combination of two or more kinds thereof in an optional combination and ratio.

Examples of the β-crystallizing agent include amide compounds; tetraoxaspiro compounds; quinacridones; iron oxides having a nanoscale size; alkaline metal salts or alkaline earth metal salts of carboxylic acids typified by potassium 1,2-hydroxystearate, magnesium benzoate, magnesium succinate, or magnesium phthalate; aromatic sulfonic acid compounds typified by sodium benzenesulfonate or sodium naphthalenesulfonate; diesters or triesters of dibasic carboxylic acids or tribasic carboxylic acids; phthalocyanine pigments typified by phthalocyanine blue; two-component system compounds consisting of a component A which is an organic dibasic acid and a component B which is an oxide, a hydroxide, or a salt of a metal of Group 2 of the periodic table; compositions consisting of cyclic phosphorus compounds and magnesium compounds; and the like.

Among these β-crystallizing agents, the amide compound is preferable. The piezoelectric characteristics can be improved by using the amide compound in the electret film. Examples of the amide compound include N,N'-dicyclohexyl-2,6-naphthalenedicarboxylic amide, N,N'-dicyclohexylterephthalamide, N,N'-diphenylhexanediamide, and the like. Among the compounds, N,N'-dicyclohexyl-2,6-naphthalenedicarboxylic amide is preferable. The amide compound has an amide group having high polarity. Therefore, it is considered that a charge can be localized in a crystal structure, and the amide compound has high piezoelectric characteristics.

On the other hand, the compound having high polarity, such as the amide compound, has a problem in that the compound has poor dispersibility due to electrostatic interaction with a polypropylene-based resin having low polarity and is likely to aggregate. However, the general β-crystallizing agent has a characteristic in which the general β-crystallizing agent is dissolved in the polypropylene-based resin in a certain temperature range. With such a characteristic, the β-crystallizing agent is uniformly dispersed in the polypropylene-based resin, and the crystal derived from the β-crystallizing agent is easily uniformly precipitated. Therefore, it is considered that the crystals of the amide compound having high polarity are uniformly dispersed in the polypropylene-based resin having low polarity, and thus the piezoelectric characteristics can be improved.

Specific examples of the commercially available β-crystallizing agent include β-crystallizing agent "NUJESTER NU-100" manufactured by New Japan Chemical Co., Ltd., and specific examples of the propylene-based resin to which the β-crystallizing agent is added include polypropylene "Bepol B-022SP" manufactured by Aristech Chemical Corporation, "Beta (β)-PP BE60-7032" manufactured by Borealis AG, polypropylene "BNX BETAPP-LN" manufactured by Mayzo, Inc., and the like.

The content of the β-crystallizing agent in the electret film according to the present embodiment can be appropriately adjusted in accordance with the type of the β-crystallizing agent, the composition of the polypropylene-based resin, or the like. However, with respect to 100 parts by mass of the polypropylene-based resin, the content is preferably 0.0001 parts by mass or more, more preferably 0.001 parts by mass or more, and yet more preferably 0.01 parts by mass or more. In addition, the content is preferably 5.0 parts by mass or less, preferably 3.0 parts by mass or less, preferably 1.0 parts by mass or less. In a case where the content of the β-crystallizing agent is 0.0001 parts by mass or more with respect to 100 parts by mass of the polypropylene-based resin, the β-crystal is sufficiently generated and grown in the polypropylene-based resin during production, and sufficient β-crystal activity can be ensured. Thus, sufficient β-crystal activity can be ensured even in a case where the polypropylene-based resin is formed into a porous film. Therefore, a porous electret film having desired piezoelectricity is obtained by performing the charging treatment on the porous film. On the other hand, in a case where the content of the β-crystallizing agent is 5.0 parts by mass or less with respect to 100 parts by mass of the polypropylene-based resin, there is an advantage in terms of economy. It is preferable that the β-crystallizing agent not bleed onto the surface of the film.

(1-1-3) Other Components

In a case where the electret film according to the embodiment of the present invention is a porous film, a foaming agent, a filler, or the like may be included in addition to or together with the above-mentioned β-crystallizing agent.

For example, in a case where a film which has been made porous by chemical or physical foaming is used as the electret film according to the embodiment of the present invention, it is preferable to add a chemical foaming agent, a physical foaming agent, a supercritical fluid, a thermally expandable microcapsule, or the like to the resin which is a main component. The components may be used alone or in combination of two or more kinds thereof.

Further, in a case where a film which has been made porous by stretching is used as the electret film according to the embodiment of the present invention, it is also preferable to add an immiscible resin or an inorganic filler to the resin, which is a main component. Examples of the inorganic filler include calcium carbonate, calcium sulfate, barium carbonate, barium sulfate, titanium oxide, talc, clay, kaolinite, montmorillonite, and the like.

The electret film in the present embodiment may appropriately include various additives such as, for example, a heat stabilizer, an antioxidant, an ultraviolet absorber, a light stabilizer, a crystallizing agent, a colorant, an antistatic agent, a hydrolysis inhibitor, a lubricant, a flame retardant, a conductive agent, an elastomer, and the like, as long as the properties of the electret film are not impaired.

The porosity of the electret film in the present embodiment is usually 0% or more, preferably 5% or more, more preferably 10% or more. In addition, the porosity is preferably 70% or less, more preferably 50% or less, yet more preferably 40% or less, and even more preferably 30% or less.

In a case where the porosity of the electret film is the upper limit or less, the pores are less likely to collapse, and thus a breakdown voltage resistance is improved. Further, in a case where the porosity of the electret film is the above-mentioned lower limit or more, the piezoelectric characteristics can be further improved. The porosity of the electret film is calculated by the method to be described later.

The thickness of the electret film in the present embodiment is preferably 10 μm or more, more preferably 15 μm or more, and yet more preferably 20 μm or more. In addition, the thickness is preferably 1,000 μm or less, more preferably 750 μm or less, and yet more preferably 500 μm or less.

By setting the thickness of the electret film to be within the above-mentioned range, the piezoelectric characteristics are improved without unnecessarily increasing the thickness of the piezoelectric sensor. It should be noted that the thickness of the electret film is measured by a method to be described later.

(1-2) Electrode

FIG. 2 is an exploded perspective view showing a basic configuration of the sensor 10 used in the first embodiment of the present invention. As shown in FIG. 2, the sensor 10 preferably includes an electret film 11 and at least one pair of electrodes 12a and 12b. As shown in FIG. 2, it is preferable that the pair of electrodes 12a and 12b be provided to sandwich the electret film 11. The pair of electrodes 12a and 12b are respectively provided with signal extraction lines 13a and 13b which output signals (voltages) from the electrode 12a or 12b. The signal extraction lines 13a and 13b respectively function as the output terminals T11 and T12 shown in FIG. 1.

The electrodes 12a and 12b may have conductivity, and may suitably employ aluminum foil, copper foil, silver foil, gold foil, nickel foil, tin foil, a carbon sheet, or the like.

The structures of the signal extraction lines 13a and 13b are not particularly limited, but each preferably has a structure in which a conductive wire is coated with an insulating medium. It is preferable that the conductive wire be made of a conductor such as copper, aluminum, and the like. Examples of the material of the insulating medium include ethylene tetrafluoride/ethylene copolymer (ETFE), vinyl chloride, crosslinked polyethylene, and the like. Further, the diameters and lengths of the signal extraction lines 13a and 13b are not particularly limited, and can be appropriately selected in accordance with the size of the sensor 10, the type of the electrodes 12a and 12b, and the like.

The thickness of each of the electrodes 12a and 12b is preferably 2 $\mu$m or more, more preferably 3 $\mu$m or more, and yet more preferably 5 $\mu$m or more. In addition, the thickness is preferably 100 $\mu$m or less, more preferably 75 $\mu$m or less, and yet more preferably 50 $\mu$m or less.

The thickness of the electrodes 12a and 12b is 2 $\mu$m or more. Therefore, the electrodes 12a and 12b are able to exhibit conductive stability. On the other hand, the thicknesses of the electrodes 12a and 12b are 100 $\mu$m or less. Therefore, the flexibility of the sensor 10 can be increased.

As described above, in the present embodiment, the sensor 10, which is a piezoelectric element-type sensor, and the first detection circuit 20, which detects the continuous pressure applied to the sensor 10 from the output voltage of the sensor 10, are provided. Here, the first detection circuit 20 has a capacitor 21 that is connected in parallel to the sensor 10 and a high-input impedance circuit 22 that is connected in series to the sensor 10. Thereby, in the first detection circuit 20, so to speak, a signal obtained by integrating the pulsed signal which is output from the sensor 10 can be obtained. Thereby, it is possible to acquire the static information with one piezoelectric element-type sensor which is less expensive than the load cell.

[Second Embodiment]

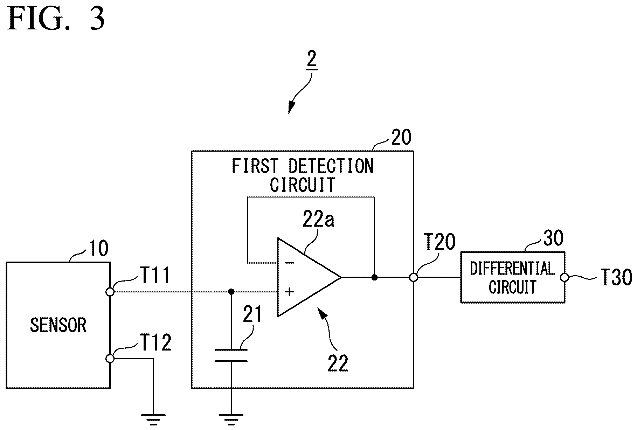

FIG. 3 is a block diagram showing a configuration of a main part of an electronic apparatus according to a second embodiment of the present invention. It should be noted that, in FIG. 3, the same configurations as those shown in FIG. 1 are denoted by the same reference numerals. As shown in FIG. 3, the electronic apparatus 2 according to the present embodiment has a configuration in which a differential circuit 30 is added to the electronic apparatus 1 shown in FIG. 1. The electronic apparatus 2 is configured to acquire the dynamic information in addition to the static information with one piezoelectric element-type sensor.

The differential circuit 30 is connected to the output terminal T20 of the first detection circuit 20. That is, the differential circuit 30 is connected in series to the first detection circuit 20. The differential circuit 30 is a circuit that detects a change in pressure applied to the sensor 10 by performing time differentiation on the signal which is output from the output terminal T20 of the first detection circuit 20. That is, the differential circuit 30 obtains the dynamic information from the detection result (the static information) of the first detection circuit 20. The differential circuit 30 outputs the detection result from the output terminal T30. Any well-known differential circuit can be used as the differential circuit 30. The differential circuit 30 may be an analog circuit or a digital circuit.

Here, the signal which is output from the output terminal T20 of the first detection circuit 20 is, so to speak, a signal obtained by integrating the pulsed signal which is output from the sensor 10 (static signal). On the other hand, the signal which is output from the output terminal T30 of the differential circuit 30 is a signal (dynamic signal) obtained by differentiating the signal which is output from the output terminal T20 of the first detection circuit 20. Therefore, in the present embodiment, the static information and the dynamic information can be acquired with one piezoelectric element-type sensor.

As described above, in the present embodiment, in addition to the sensor 10 and the first detection circuit 20, the differential circuit 30 is provided, which is connected in series to the first detection circuit 20 and which detects the change in pressure applied to the sensor 10 by differentiating the detection result of the first detection circuit 20. Therefore, the static information and the dynamic information can be acquired with one piezoelectric element-type sensor.

[Third Embodiment]

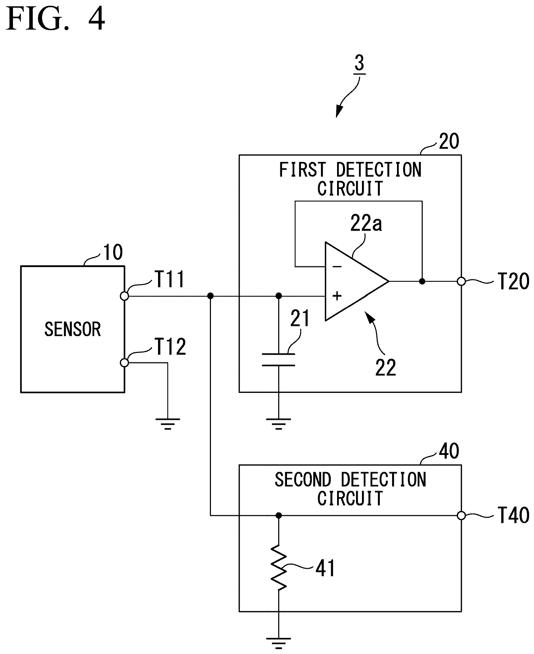

FIG. 4 is a block diagram showing a configuration of a main part of an electronic apparatus according to a third embodiment of the present invention. It should be noted that, in FIG. 4, the same configurations as those shown in FIG. 1 are denoted by the same reference numerals. As shown in FIG. 4, an electronic apparatus 3 according to the present embodiment has a configuration in which a second detection circuit 40 is added to the electronic apparatus 1 shown in FIG. 1. The electronic apparatus 3 is configured to acquire the dynamic information in addition to the static information with one piezoelectric element-type sensor, as in the electronic apparatus 2 shown in FIG. 3.

The second detection circuit 40 is a circuit including a resistor 41, and detects a change in pressure applied to the sensor 10 from the output voltage of the sensor 10. That is, the second detection circuit 40 detects the dynamic information from the output voltage of the sensor 10. The second detection circuit 40 is connected to the output terminal T11 of the sensor 10, in the same manner as the first detection circuit 20. That is, the first detection circuit 20 and the second detection circuit 40 are connected in parallel to each other.

The resistor 41 of the second detection circuit 40 is connected in parallel to the sensor 10. Specifically, one end of the resistor 41 is connected to the output terminal T11 of the sensor 10, and the other end thereof is grounded. One end (end portion connected to the output terminal T11 of the sensor 10) of the resistor 41 is connected to an output terminal T40 of the second detection circuit 40. The second detection circuit 40 outputs the detection result from the output terminal T40. The input impedance of the second detection circuit 40 is, for example, 0.1 M$\Omega$ or more and 10 M$\Omega$ or less.

The input impedance of the high-input impedance circuit 22 provided in the first detection circuit 20 is set to be higher than the input impedance of the second detection circuit 40. Specifically, the input impedance of the high-input impedance circuit 22 is 104 or more and 108 times or less than the input impedance of the second detection circuit 40. For example, in a case where the input impedance of the second detection circuit 40 is 0.1 M$\Omega$, the input impedance of the high-input impedance circuit 22 is set to be 1 G$\Omega$ or more and 10 T$\Omega$ or less. Further, for example, in a case where the input impedance of the second detection circuit 40 is 10 M$\Omega$, the input impedance of the high-input impedance circuit 22 is set to be 0.1 T$\Omega$ or more and 1 PT$\Omega$ or less.

Here, the signal which is output from the output terminal T20 of the first detection circuit 20 is, so to speak, a signal obtained by integrating the pulsed signal which is output from the sensor 10 (static signal). On the other hand, the signal which is output from the second detection circuit 40 is the same signal (dynamic signal) as the pulsed signal which is output from the sensor 10. Therefore, in the present embodiment, the static information and the dynamic information can be acquired with one piezoelectric element-type sensor.

As described above, in the present embodiment, in addition to the sensor 10 and the first detection circuit 20, the second detection circuit 40 is provided, which detects the change in pressure applied to the sensor 10 from the output voltage of the sensor 10. Therefore, the static information and the dynamic information can be acquired with one piezoelectric element-type sensor.

[Fourth Embodiment]

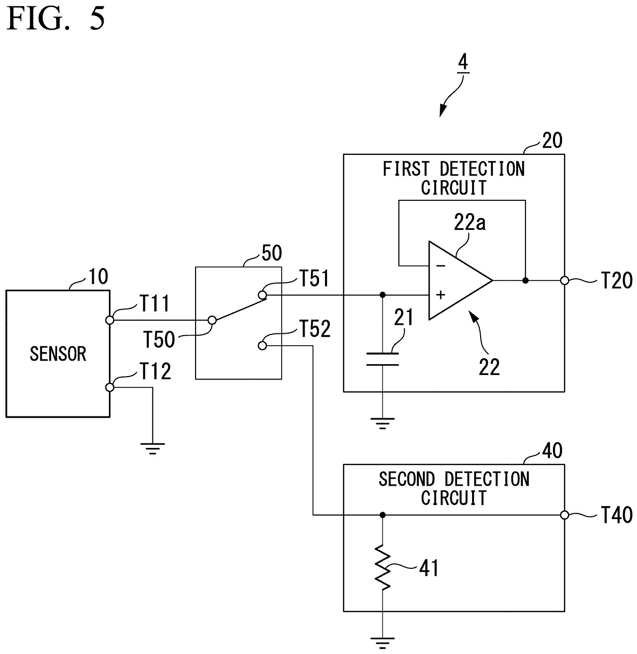

FIG. 5 is a block diagram showing a configuration of a main part of an electronic apparatus according to a fourth embodiment of the present invention. In FIG. 5, the same configurations as those shown in FIG. 4 are denoted by the same reference numerals. As shown in FIG. 5, the electronic apparatus 4 according to the present embodiment has a configuration in which a switching circuit 50 is added to the electronic apparatus 3 shown in FIG. 4. The electronic apparatus 4 is configured to acquire the dynamic information in addition to the static information with one piezoelectric element-type sensor, as in the electronic apparatus 3 shown in FIG. 4, but is configured to selectively acquire the static information and the dynamic information.

The switching circuit 50 is a circuit including one input terminal T50 and two output terminals T51 and T52, and connects the input terminal T50 to the output terminal T51 or the output terminal T52. The input terminal T50 of the switching circuit 50 is connected to the output terminal T11 of the sensor 10, the output terminal T51 is connected to the first detection circuit 20, and the output terminal T52 is connected to the second detection circuit 40. The switching circuit 50 is able to connect the first detection circuit 20 to the sensor 10 to acquire the static information or connect the second detection circuit 40 to the sensor 10 to acquire the dynamic information.

As described above, in the present embodiment, in addition to the sensor 10, the first detection circuit 20, and the second detection circuit 40, the switching circuit 50 is provided, which performs switching as to whether to connect the first detection circuit 20 or to connect the second detection circuit 40 to the sensor 10. Therefore, the piezoelectric element-type sensor 1 is able to selectively acquire the static information and the dynamic information.

The electronic apparatus described above can be used as a bed sensor or a sensor for a shelf. In a case where the electronic apparatus is used as a bed sensor, for example, by disposing the film-like sensor 10 on a lower side of a mattress of the bed, it is possible to measure the static information, which is a constant pressure, and the dynamic information, which is vital data (respiration, pulse rate, body movement, and the like) of the subject. Further, in a case where the electronic apparatus is used as the sensor for a shelf, for example, the film-like sensor 10 is disposed on the shelf. Therefore, it is possible to measure the weight (static information) of the article placed on the shelf and the weight change (dynamic information) of the article.

[Examples]

Hereinafter, the electronic apparatus according to the present invention will be described in more detail with reference to Examples 1 and 2, but the present invention is not limited by the following examples as long as the present invention does not depart from the gist of the present invention. In Examples 1 and 2, cases where two types of different materials as the organic polymer-based piezoelectric material were applied to the electronic apparatuses shown in the third embodiment and the fourth embodiment are compared.

< Example 1: Porous Electret Film >

A resin composition for an electret film was obtained by mixing and melt-extruding, at 280°C through a biaxial extruder, 100 parts by mass of homopolypropylene ("NOVATEC PP FY6HA", MFR: 2.4 g/10 min [230°C, 2.16 kg load], Mw/Mn = 3.2, manufactured by Japan Polypropylene Corporation) as a polyolefin-based resin as a material of the porous electret film, which is an organic polymer-based piezoelectric material, 0.2 parts by mass of N,N'-dicyclohexyl-2,6-naphthalenedicarboxamide ("NU-100", manufactured by New Japan Chemical Co., Ltd.) as a β-crystallizing agent, and 0.1 parts by mass of a 1:1 mixture between tris(2,4-di-t-butylphenyl) phosphite and tetra-kis [3-(3',5'-di-t-butyl-4'-hydroxyphenyl)propionic acid] pentaerythritol ("IRGANOX-B225", manufactured by BASF SE) as an antioxidant. The resin composition for the electret film was put into an extruder connected to a T-die having a lip opening of 1 mm to be molded, and was guided to a cast roll to obtain a non-porous film material having a thickness of 300 $\mu$m. Thereafter, the film was stretched horizontally 7 times the original size thereof at an elongation temperature of 100°C using a film tenter (manufactured by Kyoto Machinery Co., Ltd.) to obtain a porous film.

The obtained porous film was placed on an earth plate, and using a wire electrode, a voltage of -11 kV was applied at an inter-electrode distance of 20 mm to perform the charging treatment, thereby obtaining a porous electret film.

The thickness of the obtained porous electret film was 55 $\mu$m, the porosity thereof was 20%, and the β-crystal generative capacity thereof was 92%.

The thickness thereof was measured at 10 points at random using a dial gauge of 1/1000 mm, and an average value thereof was obtained.

Further, in a case where the porous electret film was cut to obtain a piece having a width of 100 mm and a length of 100 mm as a measurement sample, from an actual mass W1 of the measurement sample and a mass W0 which was obtained when the porosity calculated on the basis of the density of the resin composition for the electret film was 0%, the porosity thereof was calculated on the basis of the following expression.

$$\text{Porosity (\%)} = \{(W0-W1)/W0\} \times 100$$

The β-crystal generative capacity was determined from differential scanning calorimetry (DSC) of the porous electret film performed by the following method. As a test device, "DSC 204F1" manufactured by NETZSCH was used. First, the temperature thereof was raised from 40°C to 200°C at 10°C/min in a nitrogen atmosphere, maintained for 1 minute, and then cooled to 40°C at 10°C/min. After the temperature was maintained for 1 minute, at a melting peak observed in a case of raising the temperature again at 10°C/min, in a case where a β-crystal melting peak was set in a melting in which a peak was present in a temperature range of 145°C to 157°C and an α-crystal melting peak was set in a melting in which a peak was observed at 158°C or higher, each melting heat quantity was obtained from an area of a region surrounded by a baseline drawn with reference to a flat portion on a high-temperature side and a peak. Then, a melting heat quantity of the α-crystal was set as ΔHα and a melting heat quantity of the β-crystal was set as ΔHβ, and melting heat quantities were calculated by the following expression.

$$\beta\text{-Crystal Generative Capacity (\%)} = [\Delta H\beta/(\Delta H\alpha+\Delta H\beta)] \times 100$$

A piezoelectric element-type sensor was produced by the following method using the obtained porous electret film.

A conductive copper foil adhesive tape "E20CU" (manufactured by DIC Corporation, electrode thickness: 19 $\mu$m, adhesive layer thickness: 21 $\mu$m) having an adhesive layer on one surface was cut into a 5 mm square, and the copper foil was attached to a central portion of a 10 mm square protective film (Kapton tape, thickness: 50 $\mu$m) through the adhesive layer, thereby obtaining an electrode-attached protective film. The two electrode-attached protective films were disposed such that the two electrodes faced each other, and the porous electret film cut into a square of 6 mm on each side was disposed between the two electrodes. In such a case, the porous electret film was prevented from protruding from the protective film. Further, one signal extraction line (wrapping wire Junflon manufactured by Junkosha Inc.) having a line width of 0.56 mm was sandwiched between the protective film and the adhesive layer of the conductive copper foil adhesive tape. Subsequently, the end portions of the protective films were thermally fused to each other using a heat sealer to obtain the sensor of Example 1.

[Test 1: Dynamic Stimulus and Static Stimulus]

The sensor 10 of Example 1 was placed on the load cell, and output voltage waveforms of the first detection circuit 20 and the second detection circuit 40 in a case where the sensor was pressurized by a robot from above the sensor were acquired. It should be noted that an electrostatic capacitance of the capacitor 21 of the first detection circuit 20 was set to 1000 pF, and an input impedance of the high-input impedance circuit 22 was set to 1 TΩ. Further, an input impedance of the second detection circuit 40 was set to 1 MΩ.

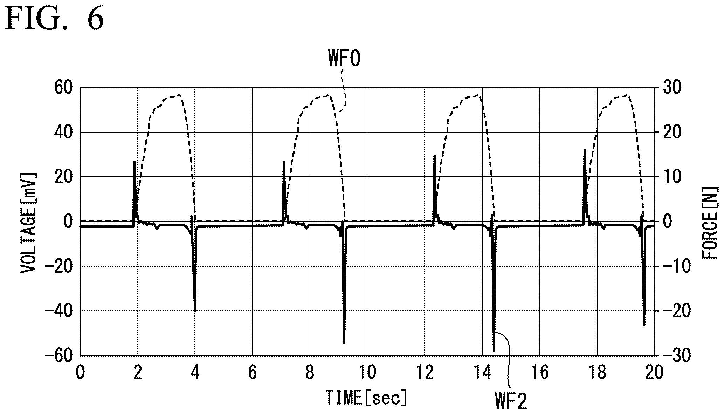

FIG. 6 is a diagram showing the waveform of the output voltage acquired by the second detection circuit 40 in a case where the sensor 10 of Example 1 was used. It should be noted that FIG. 6 also shows, in addition to a waveform WF2 of the output voltage acquired by the second detection circuit 40, an output waveform WF0 of the load cell, which indicates a state of the pressurization performed by the robot. As shown in FIG. 6, the waveform WF2 of the output signal acquired by the second detection circuit 40 is in a pulse shape, and information about the change in pressure applied to the sensor 10 (dynamic stimulus) could be verified.

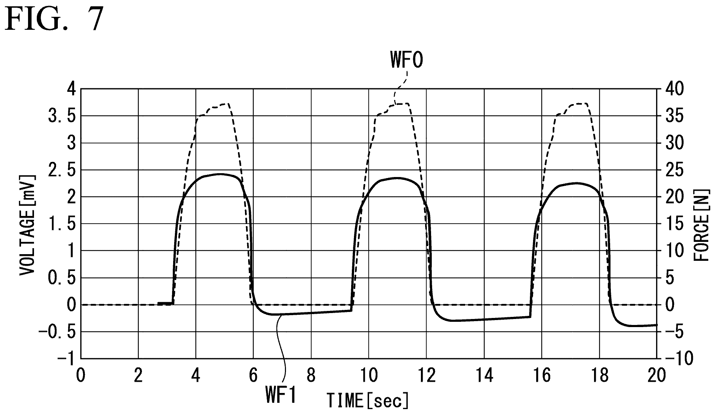

FIG. 7 is a diagram showing the waveform of the output voltage acquired by the first detection circuit 20 in a case where the sensor 10 of Example 1 was used. It should be noted that FIG. 7 also shows, in addition to the waveform WF1 of the output voltage acquired by the first detection circuit 20, the output waveform WF0 of the load cell, which indicates the state of the pressurization performed by the robot. As shown in FIG. 7, the waveform WF1 of the output signal acquired by the first detection circuit 20 (a circuit having an input impedance higher than that of the second detection circuit 40 and having the capacitor 21) was approximate to the output waveform WF0 of the load cell. Thus, it was possible to verify how long the pressure was continuously applied to the sensor 10 (static stimulation).

[Test 2: Correlation between Load and Signal Intensity]

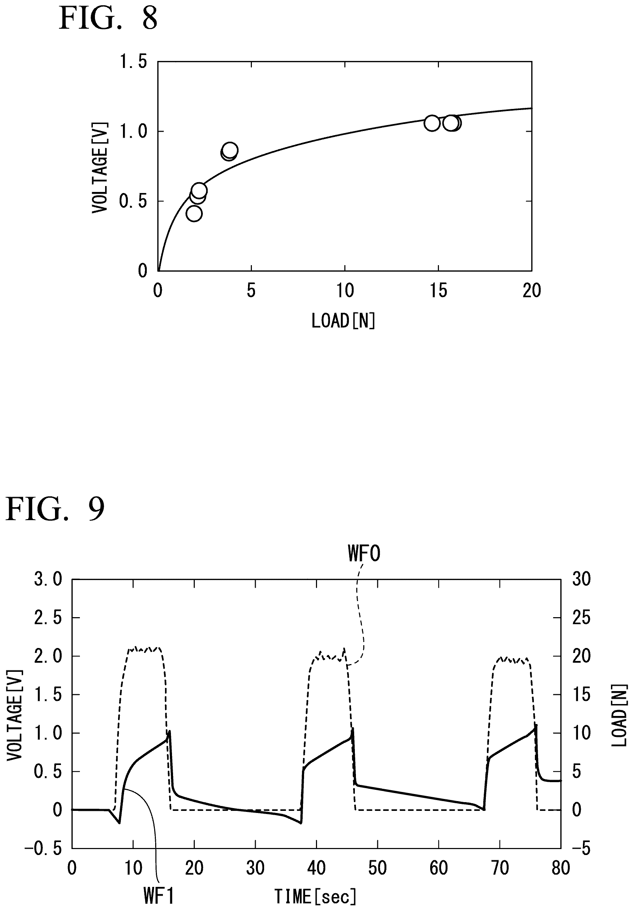

For the first detection circuit 20, the load applied to the sensor 10 was changed to acquire a signal waveform, and a correlation between the load and a signal intensity was verified. FIG. 8 is a diagram showing the relationship between the magnitude of the load applied to the sensor 10 of Example 1 and the magnitude of the output voltage of the first detection circuit 20. As shown in FIG. 8, it was verified that the signal intensity tended to increase as the load increased.

< Example 2: Polyvinylidene Fluoride Film >

A piezoelectric element-type sensor was produced by the same method as in Example 1 using a polyvinylidene fluoride film having a thickness of 80 μm (KREHA KF piezo film, manufactured by KREHA Corporation) as the organic polymer-based piezoelectric material.

[Test 3: Static Stimulation]

Regarding the polyvinylidene fluoride film, the signal waveform which was output by a normal circuit (second detection circuit 40) such as an oscilloscope was the same pulse wave as that in FIG. 6. Therefore, it was verified as to whether or not the information relating to the static stimulation could be acquired in a case where the first detection circuit 20 was formed.

FIG. 9 is a diagram showing a waveform of the output voltage acquired by the first detection circuit 20 in a case where the sensor 10 of Example 2 was used. It should be noted that, similarly to FIG. 7, FIG. 9 also shows, in addition to the waveform WF1 of the output voltage acquired by the first detection circuit 20, the output waveform WF0 of the load cell, which indicates a state of the pressurization performed by the robot. As shown in FIG. 9, the waveform WF1 of the output voltage acquired by the first detection circuit 20 is approximate to the output waveform WF0 of the load cell. Thus, it was possible to verify how long the pressure was continuously applied to the sensor 10 (static stimulation).

From the above-mentioned tests 1 to 3, it was verified that both the dynamic information and the static information can be acquired with one piezoelectric element-type sensor by combining the second detection circuit 40 and the first detection circuit 20 having an input impedance higher than that of the second detection circuit 40 and having the capacitor 21.

Further, the signal waveform obtained by the sensor 10 of Example 1 had a higher similarity to the signal waveform of the load cell than the signal waveform obtained by the sensor 10 of Example 2. Therefore, it was verified that a load applied to a sensor can be acquired with the same accuracy as that of a load cell by using a sensor including a porous electret film among piezoelectric element-type sensors.

[Industrial Applicability]

It is possible to provide an electronic apparatus that is capable of acquiring static information with one piezoelectric element-type sensor which is less expensive than a load cell, and a bed sensor and a sensor for a shelf that are provided with the electronic apparatus.

[Reference Signs List]

1 to 4: Electronic apparatus
10: Sensor
11: Electret film
20: First detection circuit
21: Capacitor
22: High-input impedance circuit
30: Differential circuit
40: Second detection circuit
50: Switching circuit

## Claims

1. An electronic apparatus comprising:

   a piezoelectric element-type sensor configured to output a voltage corresponding to an applied pressure; and
   a first detection circuit configured to detect a continuous pressure applied to the piezoelectric element-type sensor from the voltage output from the piezoelectric element-type sensor,
   wherein the first detection circuit has
   a capacitor that is connected in parallel to the piezoelectric element-type sensor, and
   a high-input impedance circuit that is connected in series to the piezoelectric element-type sensor.

2. The electronic apparatus according to Claim 1, wherein an input impedance of the high-input impedance circuit is 0.1 TΩ or more and 10 TΩ or less.

3. The electronic apparatus according to Claim 1 or 2, further comprising: a differential circuit configured to be connected in series to the first detection circuit and to detect a change in pressure applied to the piezoelectric element-type sensor by differentiating a detection result of the first detection circuit.

4. The electronic apparatus according to Claim 1 or 2, further comprising: a second detection circuit configured to detect a change in pressure applied to the piezoelectric element-type sensor from the voltage output from the piezoelectric element-type sensor.

5. The electronic apparatus according to Claim 4, wherein an input impedance of the high-input impedance circuit included in the first detection circuit is higher than an input impedance of the second detection circuit.

6. The electronic apparatus according to Claim 5, wherein the input impedance of the high-input impedance circuit included in the first detection circuit is 104 times or more and 108 times or less than the input impedance of the second detection circuit.

7. The electronic apparatus according to any one of Claims 4 to 6, wherein an input impedance of the second detection circuit is 0.1 TΩ or more and 10 TΩ or less.

8. The electronic apparatus according to any one of Claims 4 to 7, further comprising: a switching circuit configured to perform switching as to whether to connect the first detection circuit or to connect the second detection circuit to the piezoelectric element-type sensor.

9. The electronic apparatus according to any one of Claims 1 to 8, wherein an electrostatic capacitance of the capacitor is 100 pF or more and 5000 pF or less.

10. The electronic apparatus according to any one of Claims 1 to 9, wherein the piezoelectric element-type sensor includes an electret film.

11. The electronic apparatus according to Claim 10, wherein the electret film contains a polyolefin-based resin.

**12.** A bed sensor comprising: the electronic apparatus according to any one of Claims 1 to 11.

**13.** A sensor for a shelf comprising: the electronic apparatus according to any one of Claims 1 to 11.

**14.** An electronic apparatus comprising:

a piezoelectric element-type sensor configured to output a voltage corresponding to an applied pressure; and
a first detection circuit configured to detect a continuous pressure applied to the piezoelectric element-type sensor from the voltage output from the piezoelectric element-type sensor.

**15.**

The electronic apparatus according to Claim 14, wherein the first detection circuit has
a capacitor that is connected in parallel to the piezoelectric element-type sensor, and
a high-input impedance circuit that is connected in series to the piezoelectric element-type sensor.

**16.** The electronic apparatus according to Claim 14 or 15, further comprising: a differential circuit configured to be connected in series to the first detection circuit and to detect a change in pressure applied to the piezoelectric element-type sensor by differentiating a detection result of the first detection circuit.

**17.** The electronic apparatus according to Claim 14 or 15, further comprising: a second detection circuit configured to detect a change in pressure applied to the piezoelectric element-type sensor from the voltage output from the piezoelectric element-type sensor.

# FIG. 1

# FIG. 2

FIG. 3

# FIG. 4

# FIG. 5

FIG. 6

FIG. 7

# FIG. 8

# FIG. 9

# INTERNATIONAL SEARCH REPORT

| | International application No. |
|---|---|
| | **PCT/JP2023/009915** |

**A.    CLASSIFICATION OF SUBJECT MATTER**

*G01L 1/16*(2006.01)i; *A61B 5/11*(2006.01)i; *A61B 5/113*(2006.01)i
FI:    G01L1/16 G; A61B5/11 100; A61B5/113

According to International Patent Classification (IPC) or to both national classification and IPC

**B.    FIELDS SEARCHED**

Minimum documentation searched (classification system followed by classification symbols)

G01L1/16; G01L5/00-G01L5/28; A61B5/11-A61B5/113

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | 安藤正道. 新しいヒューマンマシンインターフェイスの創出を目指した圧電性L型ポリ乳酸の工業的利用に関する研究. 関西大学学術リポジトリ. Kansai University, 31 March 2014, pp. 1-188, Internet: <URL: https://doi.org/10.32286/00000127/>, in particular, section 4.5.4 (pp. 121-123), (Kansai University Institutional Repository), non-official translation (ANDO, Masamichi. Study of Industrial Use of Piezoelectric Poly-L-Lactic Acid Intended for Creation of Novel Human-Machine Interfaces.) pp. 1-188 | 1-2, 9, 14-15 |
| Y | pp. 1-188 | 2, 10-13 |
| Y | JP 2015-45967 A (SUMITOMO ELECTRIC INDUSTRIES, LTD.) 12 March 2015 (2015-03-12) paragraphs [0004], [0075], [0076] | 10-13 |
| X | WO 2017/188130 A1 (NIPPON VALQUA IND., LTD.) 02 November 2017 (2017-11-02) paragraphs [0030]-[0206], fig. 1-19 | 1, 9, 12, 14-15 |
| Y | paragraphs [0030]-[0206], fig. 1-19 | 2, 4-8, 10-11, 13, 17 |
| Y | JP 3167796 U (METSO PAPER, INC.) 19 May 2011 (2011-05-19) paragraph [0008] | 4-8, 17 |

☑ Further documents are listed in the continuation of Box C.        ☑ See patent family annex.

| * | Special categories of cited documents: |
|---|---|

\*    Special categories of cited documents:
"A"  document defining the general state of the art which is not considered to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search | Date of mailing of the international search report |
|---|---|
| **15 May 2023** | **30 May 2023** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

**INTERNATIONAL SEARCH REPORT**

International application No.

**PCT/JP2023/009915**

| C. | DOCUMENTS CONSIDERED TO BE RELEVANT | | |
|---|---|---|---|
| Category* | Citation of document, with indication, where appropriate, of the relevant passages | | Relevant to claim No. |
| X | JP 11-94707 A (SUMITOMO ELECTRIC INDUSTRIES, LTD.) 09 April 1999 (1999-04-09)<br>paragraphs [0013]-[0020], fig. 1-4 | | 14, 16-17 |
| A | WO 2017/179508 A1 (MURATA MANUFACTURING CO., LTD.) 19 October 2017 (2017-10-19)<br>paragraphs [0028]-[0059], fig. 2-7 | | 1-17 |
| A | KR 10-1380943 B1 (CNPLUS CO., LTD.) 01 April 2014 (2014-04-01)<br>paragraphs [0043]-[0076], fig. 1-16 | | 1-17 |

Form PCT/ISA/210 (second sheet) (January 2015)

**INTERNATIONAL SEARCH REPORT**
**Information on patent family members**

International application No.

**PCT/JP2023/009915**

| Patent document cited in search report | | | Publication date (day/month/year) | Patent family member(s) | | | Publication date (day/month/year) |
|---|---|---|---|---|---|---|---|
| JP | 2015-45967 | A | 12 March 2015 | (Family: none) | | | |
| WO | 2017/188130 | A1 | 02 November 2017 | (Family: none) | | | |
| JP | 3167796 | U | 19 May 2011 | US paragraph [0011] WO EP FI | 2008/0022764 2006/075056 1839023 20055020 | A1 A1 A1 A | |
| JP | 11-94707 | A | 09 April 1999 | (Family: none) | | | |
| WO | 2017/179508 | A1 | 19 October 2017 | US paragraphs [0036]-[0067], fig. 2-7 | 2019/0044527 | A1 | |
| KR | 10-1380943 | B1 | 01 April 2014 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022044701 A
- JP 2001187030 A
- JP 2008093395 A
- JP 2010051588 A
- JP 2006271501 A
- WO 2015008677 A
- JP 3739481 B

**Non-patent literature cited in the description**

- **A. ZAMBELLI et al.** *Macromol*, 1975, vol. 8, 687
- *Macromol. Chem.*, 1986, vol. 187, 643-652
- *Prog. Polym. Sci.*, 1991, vol. 16, 361-404
- *Macromol. Symp.*, 1995, vol. 89, 499-511
- *Macromol. Chem*, 1964, vol. 75, 134